# EUROPÄISCHE PATENTSCHRIFT

(11) **EP 1 096 891 B1**
(45) Veröffentlichungstag und Bekanntmachung des Hinweises auf die Patenterteilung: **21.09.2005**
(21) Anmeldenummer: 99934649.7
(22) Anmeldetag: 09.07.1999
(51) Int. Cl.: A61B 17/86, F16B 35/04

(54) **KNOCHENSCHRAUBE**
OSTEOSYNTHESIS SCREW
VIS D'OSTEOSYNTHESE

(30) Priorität: 13.07.1998 DE 19831338
(43) Veröffentlichungstag der Anmeldung: 09.05.2001
(73) Patentinhaber: Sepitec Foundation, 9490 Vaduz (LI)
(72) Erfinder: ÖSTERLE, Helmut, A-6800 Feldkirch (AT); HASLER, Guido, CH-9437 Marbach (CH); SPIRIG, Walter, CH-9428 Platz-Walzenhausen (CH)
(74) Vertreter: Menges, Rolf
(86) Internationale Anmeldenummer: PCT/EP1999/004845
(87) Internationale Veröffentlichungsnummer: WO 2000/003648

(56) Entgegenhaltungen:
- EP-A- 0 090 453
- US-A- 5 019 079
- US-A- 5 026 374
- US-A- 5 536 127

## Beschreibung

Die Erfindung betrifft eine Knochenschraube mit einem zumindest teilweise mit einem Gewinde versehenen Schaft sowie einem an wenigstens einem Ende ausgebildeten Werkzeugangriff.

Knochenschrauben dieser Art sind in vielen Ausführungsvarianten bekannt geworden. Es wurde dabei versucht, durch besondere Ausbildung des Kopfes der Schraube, einer Eindringspitze an dem dem Kopf abgewandten Ende oder durch spezielle Ausgestaltung des Gewindebereiches eine Verbesserung zu erzielen. Es wurde auch schon versucht, eine Rückdrehsicherung für die Schrauben zu erreichen, was jedoch nur im Zusammenhang mit zu befestigenden Osteosyntheseplatten und mit besonderem konstruktiven Zusatzaufwand teilweise möglich war. Eine Verdrehsicherung ist teils natürlich auch bei Knochenmarknägeln gegeben, welche mit Längsrippen oder -rillen versehen sind. Bei solchen Knochenmarknägeln ist jedoch keine Möglichkeit der axialen Sicherung gegeben. Bei Verrriegelungsnägeln hat man die Möglichkeit der axialen Sicherung.

Aus der US-A-5 026 374 ist eine orthopädisch - chirurgische Schraube bekannt, die zur Verwendung in einem Gerät zur äußeren Fixierung, z.B. einem monoaxialen Extensionsgerät, bestimmt ist. Diese bekannte Schraube hat einen gewindelosen mittleren Abschnitt, der im Querschnitt tropfenförmig ausgebildet und mit einer axial verlaufenden Schneide versehen ist. Dieser mittlere Abschnitt kommt nicht mit dem Knochen, sondern nur mit außerhalb des Knochens befindlichem, weichem Gewebe in Kontakt. Weiter hat die bekannte Schraube einen mit Außengewinde versehenen zylindrischen Endabschnitt, der zum Eindrehen in Knochenmaterial vorgesehen ist. Wenn dieser Endabschnitt in einen Knochen eingeschraubt ist, ist die Schraube nicht gegen Verdrehen gesichert.

Die vorliegende Erfindung hat sich daher zu Aufgabe gestellt, eine Knochenschraube der eingangs genannten Art zu schaffen, mit welcher in einfacher Weise sowohl eine Verdrehsicherung als auch eine Sicherung gegen selbsttätiges axiales Verschieben ermöglicht wird.

Erfindungsgemäß gelingt dies dadurch, daß der Bereich des Schaftes mit dem Gewinde und/oder ein in den Knochen eingreifender, gewindeloser Bereich des Schaftes unrund, nach Art eines Gleichdicks, z.B. trilobular, oder mehreckig ausgestaltet ist.

Durch diese erfindungsgemäße Maßnahme wird erreicht, daß die Knochenschraube eben nach Art einer Schraube eingedreht werden kann, wobei der Bereich des Schaftes mit dem Gewinde durch den Gewindeeingriff für eine axiale Sicherung gegen eine selbsttätiges Verschieben sorgt. Mit der Ausbildung des Bereiches mit dem Gewinde und/oder eines eventuell vorhandenen gewindefreien Bereiches nach der vorliegenden Erfindung wird zudem eine optimale Verdrehsicherung geschaffen. Es wird dabei aber nicht der bei Metallkonstruktionen auftretende feste Sitz der Schraube in dem Bohrloch ausgenutzt, weil eben im Bereich eines Knochens nicht diese Radialkräfte auf die Schraube einwirken können. Durch die unrund od.dgl. ausgeführte Querschnittsform des Gewindebereiches oder eines gewindefreien Bereiches wird unmittelbar nach dem Setzen der Schraube durch elastische Anpassung des hier elastisch deformierbaren Knochens von Anfang an und später durch nachwachsenden Knochen ein Formschluß zwischen dem Knochenmaterial und der Oberfläche der Knochenschraube erzielt. Die erfindungsgemäße Knochenschraube ist daher schon von Anfang an und dann zunehmend gegen das sonst bei herkömmlichen Knochenschrauben infolge repetiver Lastwechsel vorkommende selbsttätige Ausdrehen gesichert. Es wird also ein Formschluß zwischen der Knochenschraube und dem Knochen geschaffen, der eine Verdrehsicherung für die Schraube bewirkt.

Die erfindungsgemäßen Maßnahmen sind bei allen in der Medizintechnik (Human- und Veterinärmedizin) möglichen Einsätze von Knochenschrauben vorteilhaft anwendbar. Es bedarf für die Verdrehsicherung durch den Formschluß zwischen Knochen und Knochenschraube keiner großen Kräfte. Es ist daher die einmal eingedrehte Knochenschraube in optimaler Weise gegen Verdrehen und infolge des Vorhandenseins eines Gewindes auch gegen Verschieben in axialer Richtung gesichert.

Trotzdem ist aber ein nachträgliches Lösen einer solchen Schraube in einfacher Weise möglich. Der nachwachsende Knochen gibt beim Aufbringen eines schon kleinen Drehmomentes auf die Knochenschraube nach, so daß diese ohne weiteres wieder herausgedreht werden kann, falls dies erforderlich ist. Durch die unrunde Ausbildung wird lediglich erreicht, daß sich die Knochenschraube nicht selbsttätig löst und somit herausdrehen kann. Es bedarf also für die Lagesicherung gegen Verdrehen und gegen axiales Verschieben nur geringer Kräfte.

Um die Knochenschraube in üblicher Weise leicht in ein vorgebohrtes Loch eindrehen zu können, wird vorgesehen, daß das Gewinde auf dem Schaft selbstfurchend und/oder selbstschneidend ausgebildet ist. Die Knochenschraube wirkt also immer selbstformend bezüglich der Herstellung des Gewindes in dem Bohrloch.

Ausführungsbeispiele der Erfindung werden in der nachstehenden Beschreibung anhand der Zeichnung noch näher erläutert. Es zeigen:
Fig.1 eine Knochenschraube in Seitenansicht;
Fig.2 einen Schnitt nach der Linie II-II in Fig.1 oder nach der Linie II-II in Fig.4;
Fig.3 einen Schnitt nach der Linie III-III in Fig.4;
Fig.4 eine zweite Ausführungsform einer erfindungsgemäßen Knochenschraube.

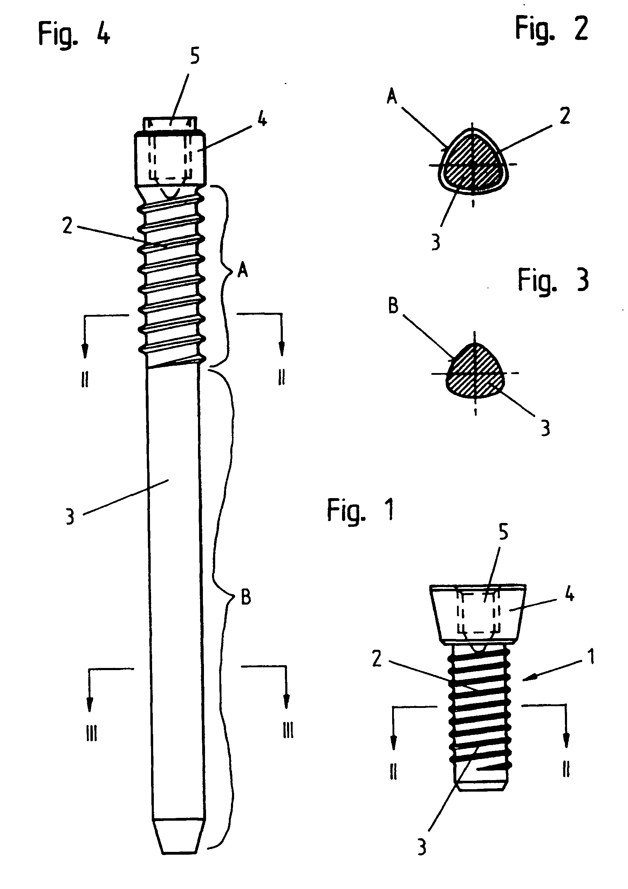

Bei einer Knochenschraube 1 mit einem mit einem Gewinde 2 versehenen Schaft 3 sowie einem an wenigstens einem Ende ausgebildeten Kopf 4 mit einem Werkzeugangriff 5 ist der Schaft 3 und somit auch das Gewinde 2 trilobular ausgeführt. Solche trilobulare Gewinde 2 (siehe auch der Schnitt gemäß Fig.2) werden in der Maschinenbautechnik zur Herstellung eines Gewindes in einem Bohrloch eingesetzt. Bei Knochenschrauben werden solche Ausbildungen nicht eingesetzt, da der Fachmann weiß, daß die Herstellung des Gewindes in einem vorgebohrten Loch in einem Knochen infolge der Konsistenz des Knochenmaterials kein Problem darstellt. Hier wird dieses trilobulare Gewinde auch nicht primär benötigt, um eben das Gewinde herzustellen, sondern um eine Verdrehsicherung der Knochenschraube zu bewirken, weil das schnell nachwachsende Gewebe sofort und mit der Zeit auch das langsamer nachwachsende Knochenmaterial diesen unrunden Querschnitt der Knochenschraube formschlüssig umgibt. Die gebräuchlichste Form bei der Ausbildung eines Gewindes dieser Art ist ein Querschnitt nach Art eines Gleichdicks und hier insbesondere die trilobulare Form.

Im Rahmen der Erfindung ist jede Art einer unrunden Ausbildung des Gewindebereiches oder des gewindefreien Bereiches, eine mehreckige Ausgestaltung oder auch jede andere Form nach Art eines Gleichdicks möglich, wobei zusätzlich zu diesen Querschnittsformen auch Erhebungen und/oder Vertiefungen vorgesehen werden können. Es ist dabei gleichgültig, für welchen Einsatzbereich in der Medizintechnik diese Schrauben eingesetzt werden. Eine erfindungsgemäße Ausbildung ist sowohl bei Knochenschrauben aus Metall, z.B. rostfreiem Stahl, Titan, CoCr-Legierungen oder geeigneten Edelmetallen, als auch aus gegebenenfalls glasfaserverstärkten Polymeren oder aus Keramik einzusetzen. Das Gewinde 2 auf der Knochenschraube 1 kann selbstschneidend und/oder selbstfurchend, also in irgend einer Weise gewindeselbstformend ausgebildet sein.

Bei der Ausführung nach Fig.4 ist eine Knochenschraube 1 vorgesehen, welche einen Bereich A mit einem Gewinde 2 und einen gewindefreien Bereich B aufweist. Für spezielle Einsätze sind solche Knochenschrauben erforderlich, wobei nur ein relativ kurzer Bereich A mit einem Gewinde 2 notwendig ist, um eben die axiale Halterung der Schraube zu bewirken. Die gesamte Länge des Schaftes 3 ist nur notwendig, um die Knochen in der gewünschten Lage ausgerichtet zu halten. Hier kann nur der Bereich A mit dem Gewinde 2 und/oder der gewindefreie Bereich B des Schaftes 3 (siehe auch Darstellung in Fig.3) unrund od.dgl. ausgeführt werden. Es muß lediglich durch die spezielle Ausbildung eines Bereiches A und/oder B des Schaftes 3 die Verdrehsicherheit in Zusammenwirken mit dem nachwachsenden Knochen gewährleistet sein. Somit ist es auch denkbar, daß das Gewinde 2 und der Schaft 3 in diesem Bereich A zylindrisch ausgeführt sind. Der ein- oder beidseitig an den Bereich A mit dem Gewinde 2 anschließende gewindefreie Bereich B des Schaftes 3 ist dann unrund, nach Art eines Gleichdicks, z.B. trilobular, oder mehreckig ausgestaltet. Es kann also auch eine Knochenschraube eingesetzt werden, bei der ein bisher übliches Gewinde eingesetzt wird, wobei dann jedoch ein in den Knochenbereich eingreifender gewindefreier Bereich unrund od.dgl. ausgeführt ist. In einem solchen Falle bewirkt das Gewinde die Sicherung gegen ein axiales Verschieben der Knochenschraube und der im Querschnitt unrund od.dgl. ausgeführte gewindefreie Bereich des Schaftes bewirkt durch den Formschluß mit dem nachwachsenden Knochen die Verdrehsicherung.

Im Rahmen der Erfindung wäre es auch möglich, auf einem Teilabschnitt oder über die ganze Länge des gewindefreien Bereiches B des Schaftes 3 umlaufende radiale Rillen und/oder Rippen auszubilden. Diese Rillen und/oder Rippen können zusätzlich zur Verdrehsicherung und zur Axialsicherung beitragen. Der Bereich A mit dem Gewinde 2 kann auch im Mittelbereich oder mit kleinerem Abstand vom Kopf 5 der Knochenschraube ausgebildet sein, so daß dann beidseitig des Bereiches A mit dem Gewinde 2 gewindefreie Bereiche B vorgesehen wären. Es kann dann auch nur ein solcher gewindefreier Bereich B unrund od.dgl. ausgeführt sein. Es wäre auch denkbar, die Knochenschraube 1 für spezielle Einsatzzwecke selbstbohrend auszuführen, also mit am freien Ende ausgebildeter Eindringspitze oder mit einem entsprechenden Bohrteil.

Femer wäre es möglich, neben den Bereichen A oder B des Schaftes oder anstelle dieser Bereiche den Bereich des Kopfes einer Knochenschraube unrund auszubilden. Eine solche Ausgestaltung ist insbesondere im Zusammenwirken mit Osteosyntheseplatten oder aber bei versenkter Anordnung des Kopfes der Knochenschraube sinnvoll und vorteilhaft.

## Patentansprüche

1. Knochenschraube mit einem zumindest teilweise mit einem Gewinde versehenen Schaft sowie einem an wenigstens einem Ende ausgebildeten Werkzeugangriff, **dadurch gekennzeichnet, daß** der Bereich (A) des Schaftes (3) mit dem Gewinde (2) und/oder ein in den Knochen eingreifender gewindeloser Bereich (B) des Schaftes (3) unrund, nach Art eines Gleichdicks, z.B. trilobular, oder mehreckig ausgestaltet ist.

2. Knochenschraube nach Anspruch 1, **dadurch gekennzeichnet, daß** das Gewinde (2) auf dem Schaft (3) selbstfurchend und/oder selbstschneidend ausgebildet ist, sind.

## Claims

1. A bone screw having a shank provided at least partly with a screw-thread and also having a tool engagement portion formed at at least one end, **characterised in that** the zone (A) of the shank (3) with the screw-thread (2) and/or a threadless zone (B) of the shank (3) engaging into the bone is of non-circular, constant-diameter type, for example trilobular, or of polygonal conformation.

2. A bone screw according to Claim 1, **characterised in that** the screw-thread (2) on the shank (3) is designed to be thread-forming and/or self-tapping.

## Revendications

1. Vis pour ostéosynthèse dotée d'une tige pourvue au moins partiellement d'un filetage ainsi que d'une prise d'outil formée sur au moins une extrémité,
**caractérisée en ce que**
la plage (A) de la tige (3) avec le filetage (2) est conçue de manière ovale sous forme orbiculaire, par exemple de manière trilobée, ou polygonale et/ou une plage (B) de la tige (3) sans filetage s'engrène dans l'os.

2. Vis pour ostéosynthèse selon la revendication 1,
**caractérisée en ce que**
le filetage (2) est conçu sur la tige (3) de manière autosillonnante et/ou autotaraudeuse.
